# EUROPEAN PATENT SPECIFICATION

(11) **EP 1 362 553 B1**
(45) Date of publication and mention of the grant of the patent: **20.10.2004**
(21) Application number: 03076344.5
(22) Date of filing: 22.05.1997
(51) Int. Cl.: A61B 8/08, A61B 8/00, G01S 5/22

(54) **Catheter tracking system**
Katheterverfolgungssystem
Système de poursuite de cathéter

(30) Priority: 11.06.1996 GB 9612199; 01.08.1996 GB 9616234
(43) Date of publication of application: 19.11.2003
(62) Divisional of application: 97108271.4
(73) Proprietor: Roke Manor Research Limited, Romsey, Hampshire SO51 0ZN (GB)
(72) Inventor: Lloyd, Peter Gregory, Salisbury, Wiltshire SP1 3WA (GB)
(74) Representative: Condon, Neil

(56) References cited:
- EP-A- 0 419 729
- WO-A-90/11722
- WO-A-92/03972
- WO-A-95/07657
- GB-A- 2 189 030
- US-A- 4 821 731
- US-A- 4 896 673
- US-A- 5 135 001

## Description

The present invention relates to catheter tracking systems, which operate to determine a position of a catheter within the human or animal body.

Electro-cardiography is a process for recording electrical signals created by a heart, using electrodes applied externally and, more particularly, electrodes positioned on tips of catheters inserted within the heart. In known endocardial catheter technology great use is made of in-theatre fluoroscopy to locate and guide the catheters to positions within the heart where measurements are required. The use of fluoroscopy has a disadvantage that inevitably theatre staff and the patient are exposed to X-ray radiation.

In order to circumvent this disadvantage, an endocardial catheter tracking system has been recently proposed, in which an AC magnetic field generated by a set of three coils mounted below the patient within the structure of a theatre table is used to track a tip of a catheter provided with a magnetic sensor. The system is complicated by the fact that the patient may move on the table and thus destroy the accuracy in the measurement of the position of the catheter tip relative to the heart. In order to overcome this problem a further catheter is used which also carries a magnetic sensor. This catheter is referred to as a reference catheter. The catheter tracking system is arranged to sense the position of the reference catheter and thereafter to determine the position of the measurement catheter. To this end, the reference catheter must be fixed somewhere in or near the heart in such a way so as to maintain its position relative to the heart. For example, this might be inserted in the coronary sinus or the right ventricular apex. The coronary sinus is a small blood vessel that has its entrance in the auricle close to the inferior vena cava and the entrance to the right ventricle. The coronary sinus is of varying diameter but can be as much as 10 mm. The right ventricular apex is an area at the lower end of the right ventricle in the base of the heart. It is not a well-defined location and does not offer a very good fixing position for the reference catheter. As a result of a possible range of movement of the reference catheter, the known arrangement of reference and measurement catheters provides difficulties in positioning the measurement catheter accurately, in particular when in actual use in the heart.

Furthermore, the use of an additional catheter increases a risk of infections and renders the use of this known tracking system cumbersome.

In view of the drawbacks of the prior art, it is an object of the invention to provide a catheter tracking system for tracking a catheter, which provides an improvement in accuracy and detection of a position of a catheter.

The object is solved with a tracking system having the features of any of Claims 1 or 2.

The catheter tracking system may further be provided with a plurality of acoustic transducers acoustically coupled to and/or arranged on a human or animal torso for emitting acoustic signals into the torso. The system may further comprise a plurality of video cameras which operate to monitor a position of said transducers, and at least one acoustic sensor arranged on a tip of a catheter and adapted to receive the emitted acoustic signals. With such a system an absolute position of the tip of said catheter is determined on the basis of the monitored position of the transducers and a time of flight of the acoustic signals to said acoustic sensor.

In this case the time of flight of pulses, for example, ultrasonic vibrations are used to give the position of the catheter. Alternatively, ultrasonic pulses can be generated in the tip of the catheter and received by a number of transducers arranged in known positions. In the case of ultrasonics the propagation velocities in air and human tissue are very different. There is, therefore, very strong reflection and refraction of the ultrasonic energy from the air-skin interface. For this reason and if required, the sensors can also be mounted on an operating table, provided the patient is made to lie in a pool of matching gel on the table. However, the presence of air in the lungs of the patient provides a preference for the ultrasonic signals to approach from the front of the chest. Therefore, advantageously the sensors or transducers may be mounted on the outside of the patient's chest. These would be secured using, say, a sticky index-matching material or straps. They could be positioned by the surgeon to give the best propagation to the heart (or part of the heart), for example avoiding the ribs.

In the case of externally mounted sensors their positions would need to be known. Hence the sensors are tracked using a known stereo video camera arrangement. With this arrangement the position of the sensors may be determined and tracked continuously. At least two cameras would be required, which would be mounted on a stand looking down on the patient's chest. In order to track the transducers the cameras must be able to see fiducial patterns of the sensors or transducers. If necessary the transducers may be placed under a surgical cover with the fiducial patterns visible above it, the fiducial patterns and the transducers being connected by, say, press fit studs.

The transducers emit ultrasound pulses in sequence and these are received by the sensor in the tip of the catheter. Comparison of the transmitted and received signals gives a time of flight of the signal. By applying alteration techniques in accordance with these measurements the position of the tip in three dimension can be determined.

As hereinbefore mentioned, if the angle of the tip is to be measured, then the catheter may be provided with two receivers separated by a distance along the catheter. Alternatively, a number of receivers each with, say, a cardioid response could be used to enable the direction of propagation of the ultrasonic energy to be determined.

The sensors may be acoustic sensors and the transducers may be acoustic transducers and the time of propagation of the said signals may be the time of flight of acoustic signals.

For the evaluation of the obtained time of flight values, a correlation for different propagation velocities occurring in homogenous material can advantageously be used to improve the accuracy of the positional relationships obtained.

Corrections are applied to reduce the errors resulting from tissue inhomogeneity. This can be carried out by analysing the propagation times and in effect varying the assumed propagation velocity until the greatest degree of consistency is obtained for all the propagation times. Such analysis is useful when the velocity of propagation in the medium is unknown. They can be used to correct for the effects of small-scale inhomogeneity, due to blood vessels etc.

Alternatively, where a distance travelled by the acoustic signals is small, a variation in time of flight due to tissue inhomegenities may be ignored since an effect thereof introduces negligible errors.

Further advantageous modifications for carrying out the invention are depicted in the attached dependent claims.

The invention will hereinafter be described in detail by way of example only, with reference being made to the attached drawings in which;
FIGURE 1 shows a schematical diagram of an arrangement of a self referencing catheter which is not part of the present invention;
FIGURE 2 shows a schematic diagram of a further arrangement of a self referencing catheter which is not part of the present invention;
FIGURE 3 is a schematic diagram of a self referencing catheter which is not part of the present invention in use with a heart, and,
FIGURE 4 is a schematic conceptual diagram of a self referencing catheter in combination with a plurality of external sensors in use in a heart.

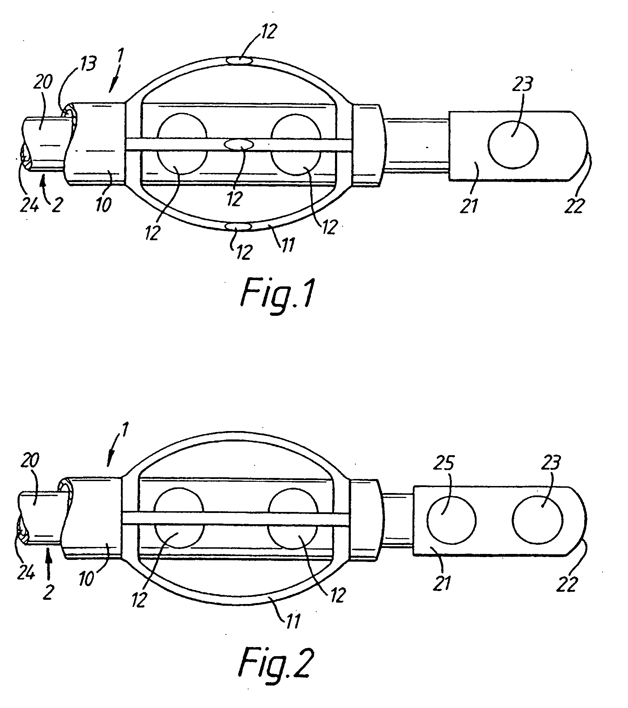

Figure 1 shows a front end of a self referencing catheter which is not part of the present invention of the invention.

According to Figure 1, there is provided an outer sheath generally referred to as 1, and a core or inner catheter, generally referred to as 2.

The outer sheath 1 has a pipe-shaped outer member or envelope 10, which has appropriate flexibility and stiffness to be inserted as a catheter into a blood vessel or the like. In the vicinity of the front end of the envelope 10, there is an extendible cage 11, which operates to increase the outer diameter of the outer sheath 1 at an end position thereof so as to be used as a fixing means for locking the end position of the outer sheath 1 at predetermined position. The cage 11, can be an inflatable cage or a mechanically operated cage. Such cages are well known in the art so that a detailed description of applicable working principles can be omitted here. It is to be noted that the cage 11, should be selected and arranged such that liquid can still pass the cage 11 in its extended state, in particular, when the outer sheath 1 is arranged and locked in a blood vessel. Further, the end portion of the outer sheath 1 is provided with acoustic sensors 12 which are arranged with a certain distance from each other. The sensors 12, are ultrasonic sensors adapted to receive an ultrasonic signal or pulse. The arrangement of the sensors 12, shown in Figure 1, is merely an example; they can be arranged on the cage 11, or at different positions on the circumference of the envelope 10.

As seen in Figure 1, the envelope 10, surrounds an inner catheter or a core 20, which is movably received in the envelope 10. The core 20, is rotatable within the envelope 10, as well as longitudinally movable therein. Advantageously, there is a certain play provided between the outer and the inner element, so as to form a free space 13, in which a wiring, piping or the like which extends along the outer sheath 1, can be received. The core 20, is advantageously a hollow body which provides an inner channel 24, for also receiving wiring or piping.

The core 20, has flexibility and stiffness properties which render it suitable to operate as a catheter. An end portion of the core 20, is provided with a head 21, with a measuring tip 22. The measuring tip 22, can be of any type for measuring electric currents, material properties or it can even operate as an optical device for visual inspection.

The measuring tip 22, can be replaced by, or combined with a means for providing a further functional tool such as a means for applying electrical current, laser light or the like. The tip can also be provided with a means for taking samples or for applying drugs, glue or the like.

According to Figure 1, the head 21 is provided with an acoustic transducer 23. In this case, an ultrasonic transducer 23, is used for emitting ultrasonic signals or pulses to be received by the ultrasonic sensors 12, on the outer sheath 10.

The working principle of the device shown in Figure 1 can be briefly described as follows:

The outer sheath 1, and the core 2, are introduced into a blood vessel or the like using well known techniques. When the portion of the outer sheath 1, provided with the cage 11, has reached a predetermined reference position, the cage 11 is extended so as to lock the outer sheath 1, at this position. The core 20, carrying the head 21, is then moved relative to the outer sheath 1 by moving within the envelope 10.

Acoustic signals are emitted by the acoustic transducer 23, arranged on the head 21. The acoustic signals are received by the acoustic sensors 12 on the outer sheath 1. Since the sensors 12 are arranged at different positions, a difference in the time of flight of a signal emitted by a single source 23, is obtained. From this difference, a position of the transducer 23, relative to the sensors 12, can be determined. Since the sensors 12, are locked to a known predetermined reference position by means of the cage 11, the actual position of the head 21, and, thus of the measuring tip 22, can be determined.

It should be noted here, that the number of sensors is not limited to two, with an increased number of sensors an even higher positional accuracy can be achieved.

Figure 2 shows an advantageous modification of the arrangement described before in detail with reference to Figure 1, which is not part of the invention. The same reference signs denote the same parts as in Figure 1, so that a repetition of this description can be omitted.

According to Figure 2, the head 21, is provided with an additional transducer 25, for emitting acoustic signals. With this arrangement, the angle of inclination of the head 21, relative to the outer sheath 1, can be determined. For this purpose, the signals emitted by the transducers 23 and 25 must be distinguishable by the sensors 12. Distinction between the signals can either be achieved by different signal characteristics, for example, by frequency, or intensity, or by a simple offset in timing for emitting the signals.

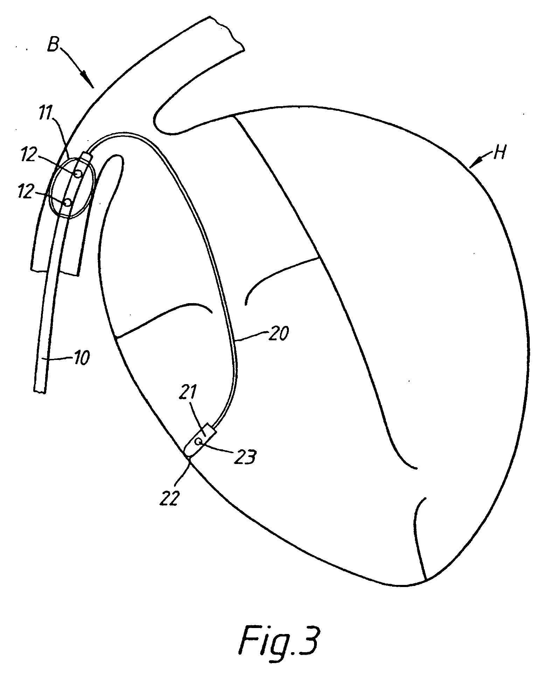

Figure 3 shows an example for an application of the catheter shown in Figure 1 in a heart. The heart H is connected to a variety of blood vessels B (e.g. vena cava or the auricle) into which the catheter is inserted in order to reach inner portions of the heart H. The reference signs used in Figure 3 denote the same parts as in Figure 1 so that a detailed description is omitted for the sake of brevity.

As can be clearly seen in Figure 3, an envelope 10, of an outer sheath of a catheter is inserted into a blood vessel B, and is locked in a predetermined or suitable reference position by means of an extendible cage 11. The head 21 fixed to a core 20 is then moved into the heart H until the measuring tip 22 has reached the desired position(s). A transducer 23, and sensors 12, co-operate in the manner described with reference to Figure 1 so as to monitor or track the actual position of the head 21 and the measuring tip 22, respectively. For this application, a synchronisation of the positional detection (i.e. emitting, receiving and evaluating signals) with the rhythm of the heartbeat is advantageous. However, this modification depends on the individual cases of application of the catheter.

It should be clear that the application to a heart is not limiting. Other organs or portions of a human or animal body can be the subject of application of the catheter tracking system. Also non-medical applications are possible, as long as a signal transfer between transducers and sensors is possible.

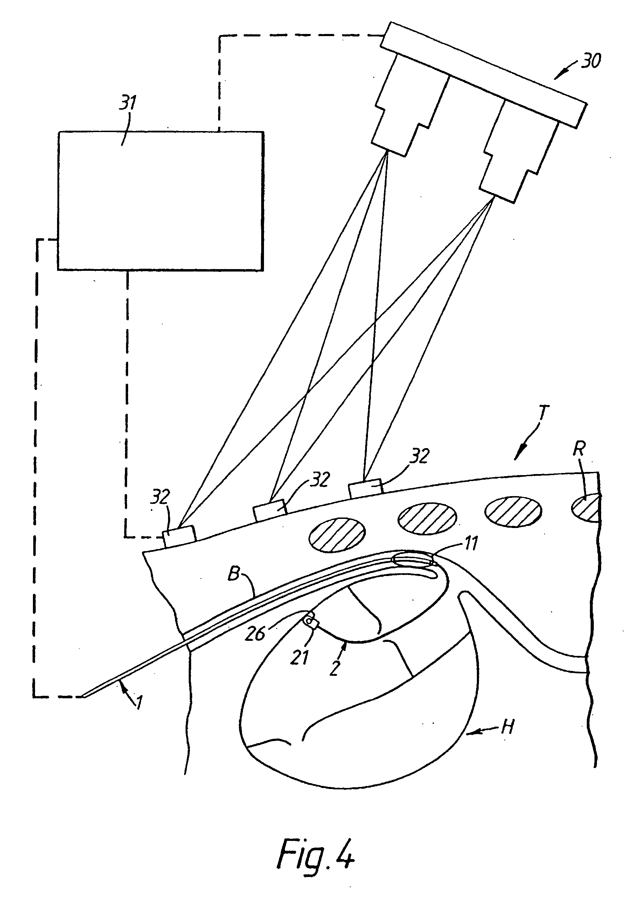

Figure 4 depicts an embodiment of the invention, applied to a human or animal heart in a torso by way of example. The same reference signs denote the same parts as in the foregoing figures, so that repetitions are omitted for the sake of brevity.

According to Figure 4, there is partly shown a torso T with ribs R beneath which a heart H is accommodated. As seen from Figure 4, a plurality of acoustic transducers 32, is arranged on the torso T. The transducers 32, are arranged such that the signals emitted therefrom pass between the ribs R. The ribs are considered opaque with respect to acoustic signals and can, additionally generate echo signals which may deteriorate the position sensing.

Above the torso T, there is arranged a stereo video-camera set 30, which is adapted to monitor the position of the transducers 32. The position of the transducers 32, is, thus , permanently known, even if the torso T is moved.

A catheter 1, 2, basically corresponding to the catheter described with reference to Figure 1 is inserted via a blood vessel B into a heart H. In contrast to the arrangements described with reference to Figures 1 to 3, the outer sheath 1, of the catheter has no acoustic sensors arranged at an end position thereof. It is also possible to refrain from using an outer sheath, so that single catheter similar to the shown inner core 2, can be used. The head 21, of the core 2, is provided with a sensor 26, for receiving acoustic signals emitted by the transducers 32. From the detected differences in time of flight of the acoustic signals emitted from the transducers 32 and received by the sensor 26, a position of the head 21, relative to the transducers 32, is obtained, which in turn is correlated with the position of the transducers 32 monitored by the camera system 30, so as to finally obtain an absolute position of the head 21. The data is processed by a data processing means 31.

According to a modification, the end position of the outer sheath 1, which is locked by means of a cage 11, as described in the foregoing embodiments, can also be provided with sensors so that a position of a reference point for the heart which moves in the torso, at least due to its heart beat, can also be monitored by means of the transducers 32, and the camera system 30.

It should be mentioned here that for simplification purposes, the transducers 32, can be replaced by sensors, whereas the sensor 26 can be replaced by a transducer forming the single source for an acoustic signal. Furthermore, the cage 11, may also be provided with sensors or transducers and arrangement of signals generated from the catheter head, the cage or externally positioned transducers may serve to provide the position of the catheter head 22, with reference to the cage and/or externally positioned transducers.

## Claims

1. A catheter tracking system, comprising a plurality of transducers (32) adapted and arranged to be in communication with a human or animal torso (T) for emitting signals into the torso (T), a plurality of video cameras (30) which operate to monitor a positional arrangement of the transducers (32), and at least one sensor (26) arranged on a tip (21) of the catheter (1, 2) and adapted to receive the emitted signals, wherein an absolute position of the tip (21) of the catheter (1, 2) is determined on a basis of the monitored arrangement of the transducers (32) and a time of propagation of the signals to the at least one sensor (26).

2. A catheter tracking system, comprising a plurality of sensors (32) adapted and arranged to be in communication with a human or animal torso (T) for sensing signals generated within the torso (T), a plurality of video cameras (30) which operate to monitor a positional arrangement of the sensors (32), and at least one transducer (26) arranged on a tip (21) of the catheter (1, 2) and adapted to generate the signals, wherein an absolute position of the tip (21) of the catheter (1, 2) is determined on a basis of the monitored arrangement of the sensors (32) and a time of propagation of the signals to the sensors (32).

3. A catheter tracking system as claimed in Claim 1, wherein the tip (21) further comprises a measurement sensor adapted for sensing electrical signals in a heart.

4. A catheter tracking system as claimed in any preceding claim, wherein the said tip (21) further comprises a means for treating adjacent tissue-in-situ.

5. A catheter tracking system as claimed in any preceding claim, wherein the said sensors and/or transducers (26, 32) are arranged in an array.

6. A catheter tracking system as claimed in any preceding claim, wherein the said tip (21) of the catheter (1, 2) is provided with an array of sensors or transducers (21) for determining an inclination angle of the said tip (21).

7. A catheter tracking system as claimed in any preceding claim, wherein the transducers (26, 32) are acoustic transducers which operate to generate acoustic signals, and wherein the sensors (32, 26) are acoustic sensors and wherein the time of propagation between the said transducers and the said sensors is the time of flight of the acoustic signals.

## Patentansprüche

1. Katheterverfolgungssystem, welches umfasst: eine Vielzahl von Wandlern (32), die so beschaffen und angeordnet sind, dass sie sich mit einem menschlichen oder tierischen Rumpf (T) in Kommunikation befinden, zum Aussenden von Signalen in den Rumpf (T), eine Vielzahl von Videokameras (30), welche in der Lage sind, eine Positionsanordnung der Wandler (32) zu überwachen, und wenigstens einen Sensor (26), der an einer Spitze (21) des Katheters (1, 2) angeordnet und so beschaffen ist, dass er die ausgesendeten Signale empfängt, wodurch eine absolute Position der Spitze (21) des Katheters (1, 2) auf der Basis der überwachten Anordnung der Wandler (32) und einer Ausbreitungszeit der Signale zu dem wenigstens einen Sensor (26) bestimmt wird.

2. Katheterverfolgungssystem, welches umfasst: eine Vielzahl von Sensoren (32), die so beschaffen und angeordnet sind, dass sie sich mit einem menschlichen oder tierischen Rumpf (T) in Kommunikation befinden, zum Erfassen von innerhalb des Rumpfes (T) erzeugten Signalen, eine Vielzahl von Videokameras (30), welche in der Lage sind, eine Positionsanordnung der Sensoren (32) zu überwachen, und wenigstens einen Wandler (26), der an einer Spitze (21) des Katheters (1, 2) angeordnet und so beschaffen ist, dass er die Signale erzeugt, wodurch eine absolute Position der Spitze (21) des Katheters (1, 2) auf der Basis der überwachten Anordnung der Sensoren (32) und einer Ausbreitungszeit der Signale zu den Sensoren (32) bestimmt wird.

3. Katheterverfolgungssystem nach Anspruch 1, wobei die Spitze (21) ferner einen Messsensor umfasst, der so beschaffen ist, dass er elektrische Signale in einem Herz erfassen kann.

4. Katheterverfolgungssystem nach einem der vorhergehenden Ansprüche, wobei die besagte Spitze (21) ferner ein Mittel zur Behandlung von angrenzendem Gewebe an Ort und Stelle umfasst.

5. Katheterverfolgungssystem nach einem der vorhergehenden Ansprüche, wobei die besagten Sensoren und/oder Wandler (26, 32) in Form einer Matrix angeordnet sind.

6. Katheterverfolgungssystem nach einem der vorhergehenden Ansprüche, wobei die besagte Spitze (21) des Katheters (1, 2) mit einer Matrix von Sensoren oder Wandlern (26, 32) zur Bestimmung eines Neigungswinkels der besagten Spitze (21) ausgestattet ist.

7. Katheterverfolgungssystem nach einem der vorhergehenden Ansprüche, wobei die Wandler (26, 32) akustische Wandler sind, welche in der Lage sind, akustische Signale zu erzeugen, wobei die Sensoren (32, 26) akustische Sensoren sind und wobei die Ausbreitungszeit zwischen den besagten Wandlern und den besagten Sensoren die Laufzeit der akustischen Signale ist.

## Revendications

1. Système de suivi de cathéter, comprenant une pluralité de transducteurs (32) adaptés et agencés pour communiquer avec un torse humain ou animal (T) pour émettre des signaux dans le torse (T), une pluralité de caméras vidéo (30) dont la fonction est de surveiller un agencement positionnel des transducteurs (32), et au moins un capteur (26) agencé sur une extrémité (21) du cathéter (1, 2) et adapté pour recevoir les signaux émis, dans lequel une position absolue de l'extrémité (21) du cathéter (1, 2) est déterminée sur la base de l'agencement surveillé des transducteurs (32) et d'un temps de propagation des signaux jusqu'au moins un capteur (26).

2. Système de suivi de cathéter, comprenant une pluralité de capteurs (32) adaptés et agencés pour communiquer avec un torse humain ou animal (T) pour détecter des signaux produits de l'intérieur du torse (T), une pluralité de caméras vidéo (30) dont la fonction est de surveiller un agencement positionnel des capteurs (32), et au moins un transducteur (26) agencé sur une extrémité (21) du cathéter (1, 2) et adapté pour produire les signaux, dans lequel une position absolue de l'extrémité (21) du cathéter (1, 2) est déterminée sur la base de l'agencement surveillé des capteurs (32) et d'un temps de propagation des signaux jusqu'aux capteurs (32).

3. Système de suivi de cathéter selon la revendication 1, dans lequel l'extrémité (21) comprend par ailleurs un capteur de mesure adapté pour détecter des signaux électriques dans un coeur.

4. Système de suivi de cathéter selon l'une quelconque des revendications précédentes, dans lequel ladite extrémité (21) comprend par ailleurs un moyen pour traiter un tissu adjacent in situ.

5. Système de suivi de cathéter selon l'une quelconque des revendications précédentes, dans lequel lesdits capteurs et/ou transducteurs (26, 32) sont agencés en réseau.

6. Système de suivi de cathéter selon l'une quelconque des revendications précédentes, dans lequel ladite extrémité (21) du cathéter (1, 2) est pourvue d'un réseau de capteurs ou transducteurs (26, 32) pour déterminer un angle d'inclinaison de ladite extrémité (21).

7. Système de suivi de cathéter selon l'une quelconque des revendications précédentes, dans lequel les transducteurs (26, 32) sont des transducteurs acoustiques dont la fonction est de produire des signaux acoustiques et dans lequel les capteurs (32, 26) sont des capteurs acoustiques et dans lequel le temps de propagation entre lesdits transducteurs et lesdits capteurs est le temps de vol des signaux acoustiques.
